# EUROPÄISCHE PATENTSCHRIFT

(11) **EP 0 970 376 B1**
(45) Veröffentlichungstag und Bekanntmachung des Hinweises auf die Patenterteilung: **09.06.2004**
(21) Anmeldenummer: 99907312.5
(22) Anmeldetag: 30.01.1999
(51) Int. Cl.: G01N 33/573, C12Q 1/527

(54) **VERFAHREN ZUM NACHWEIS VON ZENTRALNERVOSEM GEWEBE IN ERZEUGNISSEN**
METHOD FOR DETECTING A TISSUE OF A CENTRAL NERVOUS SYSTEM IN PRODUCTS
PROCEDE POUR METTRE EN EVIDENCE UN TISSU DU SYSTEME NERVEUX CENTRAL DANS DES PRODUITS

(30) Priorität: 03.02.1998 DE 19804216
(43) Veröffentlichungstag der Anmeldung: 12.01.2000
(73) Patentinhaber: ScheBo Biotech AG, 35394 Giessen (DE)
(72) Erfinder: SCHEEFERS-BORCHEL, Ursula, D-35435 Wettenberg (DE); LÜCKER, Ernst, D-35440 Linden-Forst (DE); EIGENBRODT, Erich, D-35440 Linden (DE); SCHEEFERS, Hans, D-35435 Wettenberg (DE)
(74) Vertreter: Fritzsche, Thomas M., Dr.
(86) Internationale Anmeldenummer: PCT/DE1999/000305
(87) Internationale Veröffentlichungsnummer: WO 1999/040441

(56) Entgegenhaltungen:
- WO-A-97/37227
- FR-A- 2 762 842
- LÜCKER ET AL: "Development of an integrated procedure for the detection of centrsl nervous tissue in meat producs using cholesterol and neuron specific enoilase as markers" J. FOOD PRODUCTION, Bd. 62, Nr. 3, - 1999 Seiten 268-276, XP002108976
- LÜCKER ET AL: "Procedures for the detection of unwanted ingrediants in meat products with regard to bovine spongiform encephalopathy" FLEISCHWIRTSCHAFT, Bd. 7878, Nr. 8, August 1998 (1998-08), Seiten 896-898, XP002108879

## Beschreibung

Die Erfindung betrifft ein Verfahren zum qualitativen und quantitativen Nachweis von spezifischem Risikomaterial, vorzugsweise von zentralnervösem Gewebe wie insbesonders Gehirnzusätzen sowie Zusätzen von zentralem Nervengewebe in Erzeugnissen wie Lebensmitteln und pharmazeutischen Präparaten und ein Testkit. zur Durchführung des Verfahrens.

Es ist bekannt, daß von mit Scrapie erkrankten Schafen und Ziegen stammendem Gewebe Rinderwahnsinn (Bovine spongiforme Enzephalopathie, BSE) bei Rindern erzeugt werden kann. Hierbei hat es sich gezeigt, daß der Erreger offenbar auch durch den Verzehr von infiziertem Fleisch bzw. Fleischprodukten auch auf den Menschen übertragen werden kann und dort zu einer neuen Variante der Jakob-Creutzfeldt-Erkrankung (nvCJD) führen kann. Da weiterhin gefunden wurde, daß sich die für die Auslösung der Erkrankung verantwortlichen infektiösen Proteine (Prione) besonders in den Nerven und im Gehirn ansammeln, stellen Gewebe aus diesen Organen auch unter Berücksichtigung von noch nicht entdeckten Erregern und Agenzien ein hohes Infektionsrisiko dar. Daher werden derartige von Rindern, Schafen und Ziegen stammende Organe, die üblicherweise eine hohe Prionen-konzentration enthalten, als "spezifiziertes Risikomaterial" (SRM) bezeichnet.

Rechtsvorschriften wie die Entscheidung der Kommission 97/534/EG (Amtsblatt der Europäischen Gemeinschaften Nr. L 1997, 216:95-98) sehen vor, daß derartiges spezielles, bei der Schlachtung anfallendes Risikomaterial getrennt gesammelt, eingefärbt und auch getrennt von übrigen Schlachtabfällen beseitigt werden muß. Hierzu gehören auch Augen, Mandeln oder die Milz derartiger Tiere.

Da bei der Verarbeitung von Lebensmitteln, insbesondere von Fleischerzeugnissen wie Würsten, das Zusetzen von Hirngewebe auch technische Wirkungen wie bessere Emulgierbarkeit (Brühwurstbrät) und erhöhte Stabilität (Beefburger) bietet, ist es zum Schutze des Verbrauchers notwendig, die Einhaltung des zuvor genannten Verwertungsverbotes im Rahmen der Lebensmittelüberwachung zu kontrollieren. Dabei wurden bislang von den zu untersuchenden Lebensmitteln, wie z.B. Fleischerzeugnissen histologische Präparate hergestellt, welche anschließend von besonders geschulten Fachleuten lichtmikroskopisch untersucht wurden. Mit diesem Verfahren können neben den zu erwartenden Bestandteilen der Muskulatur und Bindegewebe auch nicht erwünschte Gewebe identifiziert werden, wie Niere, Pansen oder auch Haut.

Es hat sich jedoch gezeigt, daß der Zusatz von Hirngewebe mittels lichtmikroskopischer Routinediagnostik, d. h. durch Anfertigen von 10µm Kryoschnitten und Färbung mit Picroindigocarmin/Karmalaun nicht erfaßt werden. Dies gilt auch für Ultradünnschnitte mit herkömmlicher Färbetechnik (Hämatoxylin/Eosin) sowie für Spezialfärbungen (Silberimprägnierung, Neurofibrillen, Markscheiben, Nissl-Färbung). Auch ZNS-charakteristische Bestandteile wie Hirnhaut oder Zellstrukturen wie Astrozyten sind mittels den oben genannten Verfahren nicht identifizierbar.

Es besteht daher Bedarf an einem schnellen Testverfahren, mit dem die Verwendung derartiger unerlaubter spezifizierter Risikomaterialien auch noch in geringen Mengen nachgewiesen werden kann.

Es ist bereits ein Verfahren zum Nachweis von unerwünschten Zutaten in Fleischerzeugnissen bekannt und zwar im besonderen in Hinblick auf die bovine spongiforme Enzephalophathie (BSE) [Fleischwirtschaft 77 (9), 836-840 (1997), E. Lücker und M. Bülte]. Dabei wird mit einem enzymatisch-photometrischen Verfahren der Cholesteringehalt bestimmt, wodurch sich bereits ein Zusatz von 2% Gehirngewebe in Brühwürsteu nachweisen läßt. Dieses Verfahren hat zwar den Vorteil, daß es sich auf einfache Weise mit geringen instrumentellem Zeit- und Kostenaufwand durchführen läßt. Es weist jedoch den Nachteil auf, daß es nicht möglich ist, festzustellen, ob ein erhöhter Cholesteringehalt durch verbotene Hirnzusätze oder durch Zusätze wie Eigelb, Leber oder pflanzliche 3-β-Hydroxysterine hervorgerufen wird. Daher kann er in der Praxis nur zum Auffinden von Verdachtsproben dienen.

Das glycolytische Enzym Enolase, eine 2-Phospho-D-glycerat-Hydrolase (Cras P., Martin J.J., Gheuens, J., γ-enolase and glial fibrillary acidic protein in nervous System tumors. An immunohistochemical study using specific monoclonal antibodies, Acta Neuropathol. 1988, 75:377-84 und Cras P., Soler Federsppiel, S. Gheuens J., Martin. J.-J., Lowenthal A., Demonstration of neuron-specific enolase in nonneuronal tumors using a specific monoclonal antibody, Ann. Neurol. 1986, 20:106-7) ist als Marker für neuronale und nichtneuronale Tumore bekannt.

Die Erfindung hat daher zum Ziel, den weiterhin bestehenden Bedarf an einem spezifischen, leicht durchzuführenden Verfahren zu befriedigen, mit dem spezifiziertes Risikomaterial, insbesondere Gehirn- und Nervengewebe noch bei einem geringen Gehalt zweifelsfrei nachgewiesen werden kann, und zwar auch dann, wenn das Erzeugnis bei der Zubereitung erhitzt wurde. Ein derartiges Testverfahren soll preiswert, schnell durchführbar und automatisierbar sein, damit es als Routineverfahren generell einsetzbar ist.

Dieses Ziel wird nun mittels dem erfindungsgemäßen Verfahren nach Anspruch 1 erreicht.

Es wurde nämlich gefunden, daß sich der Zusatz von Gehirnmasse bzw. zentralem Nervengewebe durch den Gehalt an γ,γ-Enolase (EC 4.2.1.11, neuronenspezifische Enolase) nachweisen läßt, wobei der Gehalt direkt proportional zur zugesetzten Gehirnmasse bzw. Nervenmasse ist. Dies ist umso überraschender, weil umfangreiche Untersuchungen der Erfinder gezeigt haben, daß in erhitzten Fleischerzeugnissen wie Brüh- und Kochwürsten mit definierten Hirnzusätzen bei der Polyacrylamid-Gelelektrophorese und beim Isoelektrischen Fokussieren (PAGIF) keine spezifischen Banden erhalten werden, die sich vom gleichen Produkt ohne Hirnzusatz unterscheiden, da sowohl die Struktur als auch die elektrochemischen bzw. proteinchemischen Eigenschaften der ZNS-spezifischen Proteine bei der Erhitzung während der Wurstherstellung (mindestens 80°C über etwa 60 Minuten) nachhaltig beeinträchtigt werden. Dies gilt auch für den Nachweis von derartigen charakteristischen Proteinen mittels immunologischer Methoden.

Erfindungsgemäß wurde nun gefunden, daß sich trotz der oben beschriebenen Proteindenaturierung ein spezifischer Nachweis von Hirngewebe bzw. zentralem Nervengewebe in Erzeugnissen bzw. Präparaten durchführen läßt. Somit ist es erfindungsgemäß möglich, derartige unzulässige Zusätze auch noch in erhitzten Fleischerzeugnissen nachzuweisen. Vorzugsweise wird der Nachweis mittels γ,γ-Enolase-spezifischen Antikörpern durchgeführt. überraschenderweise wurde festgestellt, daß sich der Nachweis auch mittels polyklonalen γ,γ -Enolase-Antikörpern spezifisch durchführen läßt..

Erfindungsgemäß wurde auch gefunden, daß sich die Empfindlichkeit des Nachweises von γ,γ-Enolase beträchtlich steigern läßt, wenn die zu untersuchende Probe vorher einer Fettextraktion unterzogen wird. Auf diese Weise ist es möglich, nach 0,25% Zusatz an Rinderhirn in Fleischerzeugnissen selektiv nachzuweisen.

Die erfindungsgemäße Fettextraktion wird mit Hilfe eines organischen Lösungsmittels durchgeführt. Vorzugsweise werden nicht-protonische apolare Lösungsmittel wie Petroleum-Benzin, Benzol, Tetrachlorkohlenstoff oder Ether verwendet. Auch die Extraktion mittels Gasen im überkritischen Zustand, wie beispielsweise CO₂ ist möglich. Optimale Bedingungen werden bei der isothermischen Extraktion im Rückfluß (Soxhlet) erreicht. Die Reduktion des Fettgehaltes sollte bezogen auf den Fettgehalt der Probe mindestens 10%, vorzugsweise mindestens 20%, im besonderen mindestens 30% bzw. bezogen auf die Probenmasse mindestens 2%, zweckmäßigerweise mindestens 5% und vorzugsweise mindestens 8% und im besonderen mindestens 15% betragen. In der Praxis hat es sich gezeigt, daß je nach Probe eine Extraktion im Bereich von 15-60% zweckmäßig ist. Vorzugsweise wird die Fettextraktion an der nativen Probe durchgeführt. Extraktion mit anderen Mitteln wie SDS oder Tensiden wie Temed oder Nonidet sind zwar möglich, jedoch werden die zuvor genannten Lösungsmittel bevorzugt. Gleiches trifft auf die Proteinanreicherung mittels umgekehrter Filtration (Zentrisart, Sartorius) zu.

Erfindungsgemäß ist es auch möglich, mittels geeigneten Antikörpern gegen Spezies-spezifische γ,γ-Enolase Hirn- und Nervenzusätze von unterschiedlichen Tierarten zu bestimmen. Dabei werden die γ,γ-Enolasen von verschiedenen Tierarten nach einer einfachen und reproduzierbaren Reinigung aus Gehirngewebe und aus Rous-Sarkomatransformierten Zellen von verschiedenen Tierarten gewonnen (Eigenbrodt, E., P. Fister, H. Rübsamen, R. R. Fries, Influence of transformation by Rous sarcome virus on the amount, phosphorylation and enzyme kinetic properties of enolase. The EMBO Journal, Vol. 2:1565-1570, 1983). Nach der Isolierung und Reinigung des so erhaltenen Enzyms kann dieses nach bekannten Verfahren zur Herstellung von poly- und monoklonalen Antikörpern verwendet werden.

Die Erfindung betrifft auch einen Testkit zur Durchführung des Verfahrens. In einer bevorzugten Ausführungsform wird dabei ein an γ,γ-Enolase bindender Antikörper, vorzugsweise ein spezifischer Antikörper mittels bekannten Verfahren an eine Festphase immobilisiert. Die zu untersuchende Probe wird dann mit dem vorzugsweise gelösten Enzym in Kontakt gebracht und in einem geeigneten Puffersystem mittels der Antikörper an die Festphase gebunden. Nach Waschen des so erhaltenen immobilisierten Antikörper-Enolase-Komplexes wird dann ein weiterer, mit einem Marker versehener sekundärer Antikörper zugesetzt, der an ein anderes Epitop der γ,γ-Enolase bindet und die Menge des Markers bestimmt. Die Menge an gebundenem Marker ist direkt proportional der Menge der Enolase in der Probe. Zweckmäßigerweise enthält der Testkit Referenzmaterial mit unterschiedlichen Hirn- und/oder Nervenzusätzen zur Sicherung der Analysenqualität.

Erfindungsgemäß ist es auch möglich, die Analytenkonzentration mittels Biosensoren zu bestimmen, wie z. B. amperometrische Sensoren, potentiometrische, ionenselektive potentiometrische oder photometrische Sensoren oder auch solche mittels Halbleiterelektroden wie Feldeffekttransistoren (FET), chemosensitive Feldeffekttransistoren (CHEMFET), suspended-gate-Feldeffekttransistoren (SGFET) oder ionensensitiven Feldeffekttransistoren. Derartige Biosensoren sind zusammenfassend in E. A. H. Hall und G. Hummel in "Biosensoren", Springer Verlag Heidelberg, Deutschland, 1995 beschrieben. Weitere Entwicklungen von ionensensitiven Feldeffekttransistoren (ISFET) oder optischen Detektoren sind unter anderem von F. Aberl und H. Wolf in "Aktuelle Trends in der Immunsensorik, Labor 2000, S. 70-96, (1993)" beschrieben. Ebenfalls geeignet ist das erfindungsgemäße Verfahren für die Durchführung mittels piezoelektrischen Schwingquarzen und Oberflächenwellenelementen, welche als Mikrowaagen verwendet werden können. Dabei wird der primäre Antikörper (der sog. Catcher) auf einem piezoelektrischen Substrat immobilisiert und nach Bindung mit der zu analysierenden γ,γ-Enolase gemessen. Derartige Sensoren sind beispielsweise von A. Leidl et al. in "Proceedings of the second international symposium on miniaturized total analyses system µTAS", Basel 1996, beschrieben. Quarzkristallmikrowaagen, wie sie von C. Köslinger et al., Fresenius J. Anal. Chem. (1994), 349, 349-354 beschrieben sind, haben sich als besonders geeignet erwiesen.

### Beispiel 1:

Es wurden Brühwürste mit definierten Zusätzen an Rinderhirn hergestellt, wie dies bei Lücker und Bülte [(1997) Fleischwirtschaft 77, Seite 136-840 (1997)] beschrieben ist. Bei diesen Brühwurststandards betrug der Anteil an Hirn 0%, 1%, 2%, 3,8%, 7,4%, 13,8% und 33,3% (BWS-a bis g). Bei einem zusätzlichen Standard wurde anstelle des Hirns 16% Eigelb zugesetzt (BWS-Ei). Die Proben wurden unter Kühlung mit Tris-Harnstoff-Puffer im Potter extrahiert (Extraktionspuffer: TRIS-Harnstoff-Puffer aus 20 mM Tris(Hydroxymethyl)-Amminomethan, 8 M Harnstoff, pH 7,6 und 15 Minuten bei 2500 UpM zentrifugiert. und zweifach filtriert. Das Filtrat wurde im Verhältnis 50:1 mit Probenpuffer (TRIS-SDS-Mercaptoethanol-Harnstoff, Probenpuffer zu gleichen Teilen aus Sammelgelpuffer, 10%igem Gew./Vol. 2-Mercaptoethanol, 10%igem Gew./Vol. SDS und 8 M Harnstoff; Sammelgelpuffer: 0,5 Tris(Hydroxymethyl)-Amminomethan, 1% Gew./Vol. SDS) verdünnt. Diese Probenextrakte können bei -18°C über mehrere Monate hindurch ohne Qualitätsverluste gelagert werden.

Die Proteinkonzentration der Extrakte wurde mit einem Proteintest von Bio-Rad (Richmond, USA) bestimmt. Die Proteine wurden mittels einer SDS-Gel-Elektrophorese mit 10%igen Acrylamidgelen nach Laemmlj [Nature, 227, 680-685 (1970)] aufgetrennt. Dabei wurde bei jeder Probe 10-100 µg Protein aufgetragen. Nach der Auftrennung wurde mit Coomassie-Blau angefärbt, wobei als Marker der Molekularmasse-Kalibrierungskit LMW von Pharmacia (Uppsala, Schweden) verwendet wurde.

Die aufgetrennten extrahierten Proteine wurden dann im Elektroblotverfahren (CTI, Idstein/Taunus) auf Nitrocellulose-Membranen (Optitran BA-S85, Schleicher und Schuell) bzw. auf PVDF-Membranen (Immobilon-P., Millipore, Bedford/USA) übertragen und mittels primärer poly- bzw. monoklonaler Antikörper in Verbindung mit sekundären Antikörpern markiert, die mit Peroxidase konjugiert waren (direkt bzw. biotinyliert). Als Substrat für die Farbreaktion wurde 3,3'-Diaminobenzidin-tetrahydrochloridhydrat (DAB, Aldrich, Milwaukee, USA) verwendet..

Als Antikörper wurden sowohl poly- als auch monoklonale Antikörper verwendet. Polyklonale Antikörper, die gegen γ,γ-Enolase gerichtet sind, z. B. Rabbit N 535 γ-Enolase, werden von Genosys hergestellt und durch IC Chemikalien GmbH, Ismaning/DE vertrieben. Ihre Kreuzreaktivität mit α-Enolase wird als <10% angegeben. Monoklonale Antikörper, die gegen die humane γ,γ-Enolase gerichtet sind, werden von DAKO, Hamburg/DE vertrieben.

Weitere erfindungsgemäß zu verwendende monoklonale Antikörper sind aus Zellinien erhältlich, die dem Mäusezellklon BBS/NC/VI-H14 entstammen. Diese monoklonalen Antikörper zeigen keine Kreuzreaktivität mit humaner α- oder β-Enolase. Soler Federsppiel, B.S., P. Cras, J. Gheueus, D. Andries, A. Lowenthal, Human γ,γ-Enolase: Two-site immunoradiometric assay with a single monoclonal antibody, Journal of Neurochemistry 1987, 48: 22-28.

### Beispiel 2:

Beispiel 1 wurde wiederholt, wobei die Wurstproben jedoch manuell mit einem Messer fein zerkleinert wurden. Von diesem gehackten Material wurden 10g auf einen Faltenfilter (Schleicher und Schuell, Nr. 597½) eingewogen und der Filter mit dem Probenmaterial in eine Extraktionshülse aus Filterpapier (Schleicher und Schnell, Nr. 603) eingebracht, mit Watte verschlossen und in einen Extraktionsapparat nach Soxhlet. Dabei wurde das 1,5-fache des Extraktionsaufsatzes an Petroleum-Benzin in den Extraktionskolben gegeben und acht Stunden unter Rückfluß extrahiert. Nach Rückdestillierung des Lösungsmittels wurden die Extraktionshülsen entnommen und bei Raumtemperatur (etwa 1 Stunde) getrocknet. Im Durchschnitt wurde ca. 36% des Fettgehaltes der Probe extrahiert.
Die Ergebnisse sind in der Tabelle angegeben.

| Tabellarische Auflistung der Ergebnisse (semiquantitativ) aus Beispiel 1 (ohne Fettextraktion) und Beispiel 2 (mit Fettextraktion) | | | | |
|---|---|---|---|---|
| Probe | Hirn-Zusatz (%) | Sonst. Zusatz (%) | Extraktion | Response |
| Brühwurst | 0 | | Ohne Fettextraktion | - |
| | 1 | | | + |
| | 2 | | | ++ |
| | 3,8 | | | +++ |
| | 7,4 | | | ++++ |
| | 13,8 | | | +++++ |
| | 33,3 | | | ++++++ |
| | 0 | Eigelb, 16% | | - |
| | 0 | | Mit Fettextraktion | - |
| | 0,25 | | | + |
| | 0,5 | | | ++ |
| | 1 | | | +++ |
| | 2 | | | ++++ |
| Kochwurst | 0 | Leber, 16% bzw. Leber, 32% | Ohne Fettextraktion | - |
| | 1 | | | - |
| | 2 | | | - |
| | 4 | | | - |
| | 8 | | | - |
| | 16 | | | - |
| | 32 | | | - |
| | 0,25 | | Mit Fettextraktion | + |
| | 0,5 | | | - |
| | 1 | | | + |
| | 2 | | | ++ |
| | 4 | | | +++ |

| | | | | |
|---|---|---|---|---|
| - Bande nicht erkennbar | | | | |
| + Bande (mit bloßem Auge) gerade erkennbar | | | | |
| ++ Bande erkennbar | | | | |
| +++ Bande gut erkennbar | | | | |
| ++++ Bande deutlich ausgeprägt | | | | |
| +++++ Bande sehr stark ausgeprägt | | | | |
| ++++++ Bande extrem ausgeprägt, in die Nachbar-Banden ausfließend | | | | |

## Patentansprüche

1. Verfahren zum qualitativen und quantitativen Nachweis von zentralnervösem Gewebe in Erzeugnissen, insbesondere in Fleischerzeugnissen, **dadurch gekennzeichnet, daß** man zugesetztes Gehirn und/oder Nervengewebe durch den Gehalt an γ,γ-Enolase in einer zu untersuchenden Probe bestimmt.

2. Verfahren nach Anspruch 1, **dadurch gekennzeichnet, daß** man den Gehalt mittels anti- γ,γ-Enolase-Antikörpern bestimmt.

3. Verfahren nach einem der vorhergehenden Ansprüche, **dadurch gekennzeichnet, daß** man die γ,γ-Enolase aus der zu untersuchenden Probe vor der Bestimmung extrahiert.

4. Verfahren nach einem der vorhergehenden Ansprüche, **dadurch gekennzeichnet, daß** man die zu untersuchende Probe lipophil extrahiert.

5. Verfahren nach einem der vorhergehenden Ansprüche, **dadurch gekennzeichnet, daß** der Antikörper ein monoklonaler Antikörper ist, der aus der Hybridoma-Zellinie BBS/NC/VI-H14 erhältlich ist.

6. Testkit zum Nachweis von Zusätzen von zentralnervösem Gewebe in Erzeugnissen, insbesondere in Fleischerzeugnissen, **dadurch gekennzeichnet, daß** er Antikörper gegen γ,γ-Enolase enthält.

7. Testkit nach Anspruch 6, **dadurch gekennzeichnet, daß** der Antikörper ein monoklonaler Antikörper, erhältlich aus der Hybridoma-Zellinie BBS/NC/VI-H14 ist.

8. Testkit nach einem der Ansprüche 6 oder 7, **dadurch gekennzeichnet, daß** er einen markierten Antikörper enthält.

9. Testkit nach einem der Ansprüche 6-8, **dadurch gekennzeichnet, daß** er einen an einer Festphase gebundenen anti- γ,γ-Enolase-Antikörper enthält.

10. Testkit nach einem der Ansprüche 6-9, **dadurch gekennzeichnet, daß** er einen Elisa- oder Biosensortest enthält.

11. Testkit nach einem der Ansprüche 6-10, **dadurch gekennzeichnet, daß** er Mittel zur lipophilen Extraktion enthält.

12. Testkit nach einem der Ansprüche 6-10, **dadurch gekennzeichnet, daß** er für die analytische Qualitätssicherung Referenzmaterial mit unterschiedlichen Hirnzusätzen enthält.

## Claims

1. Method for the qualitative and quantitative detection of central nervous tissue in products, more especially in meat products, **characterised in that** added brain and/or nerve tissue is detected by means of the content of gamma, gamma-enolase in a sample to be tested.

2. Method according to claim 1, **characterised in that** the content is determined by means of anti-gamma, gamma-enolase antibodies.

3. Method according to one of the preceding claims, **characterised in that**, before the determining process, the gamma, gamma-enolase is extracted from the sample to be tested.

4. Method according to one of the preceding claims, **characterised in that** lipophil is extracted from the sample to be tested.

5. Method according to one of the preceding claims, **characterised in that** the antibody is a monoclonal antibody, which can be obtained from the hybridoma cell line BBS/NC/VI-H14.

6. Test kit for detecting additives of central nervous tissue in products, more especially in meat products, **characterised in that** said test kit contains antibodies against gamma, gamma-enolase.

7. Test kit according to claim 6, **characterised in that** the antibody is a monoclonal antibody, obtainable from the hybridoma cell line BBS/NC/VI-H14.

8. Test kit according to one of claims 6 or 7, **characterised in that** said test kit contains a marked antibody.

9. Test kit according to one of claims 6 - 8, **characterised in that** said test kit contains an anti gamma, gamma-enolase antibody which is bound to a solid phase.

10. Test kit according to one of claims 6 - 9, **characterised in that** said test kit contains an ELISA test or a biosensor test.

11. Test kit according to one of claims 6 -10, **characterised in that** said test kit contains means for the extraction of lipophil.

12. Test kit according to one of claims 6 -10, **characterised in that** said test kit contains reference material with different brain additives for analytical quality assurance.

## Revendications

1. Procédé pour la mise en évidence qualitative et quantitative de tissus du système nerveux central dans des produits, en particulier des produits à base de viande, **caractérisé en ce qu'**on détermine la teneur en cervelle et/ou tissus nerveux ajoutés par la teneur en γ,γ-énolase dans un échantillon à contrôler.

2. Procédé selon la revendication 1, **caractérisé en ce qu'**on détermine la teneur à l'aide d'anticorps anti-γ,γ-énolase.

3. Procédé selon l'une des revendications précédentes, **caractérisé en ce qu'**on extrait la γ,γ-énolase de l'échantillon à contrôler avant le dosage.

4. Procédé selon l'une des revendications précédentes, **caractérisé en ce qu'**on extrait l'échantillon à contrôler par lipophilie.

5. Procédé selon l'une des revendications précédentes, **caractérisé en ce que** l'anticorps est un anticorps monoclonal, que l'on obtient à partir de la lignée cellulaire hybridome BBS/NC/VI-H14.

6. Kit d'essai pour la mise en oeuvre d'ajout de tissus de système nerveux central dans des produits, en particulier des produits à base de viande, **caractérisé en** qu'il comprend des anticorps anti-γ,γ-énolase.

7. Kit d'essai selon la revendication 6, **caractérisé en ce que** l'anticorps est un anticorps monoclonal que l'on peut obtenir à partir de la lignée cellulaire hybridome BBS/NC/VI-H14.

8. Kit d'essai selon l'une des revendications 6 et 7, **caractérisé en** qu'il renferme un anticorps monoclonal marqué.

9. Kit d'essai selon l'une des revendications 6 à 8, **caractérisé en** qu'il renferme un anticorps monoclonal anti-γ,γ-énolase lié à la phase solide.

10. Kit d'essai selon l'une des revendications 6 à 9, **caractérisé en** qu'il renferme un dosage ELISA ou à l'aide de biocapteurs.

11. Kit d'essai selon l'une des revendications 6 à 10, **caractérisé en** qu'il renferme des agents pour l'extraction lipophile.

12. Kit d'essai selon l'une des revendications 6 à 10, **caractérisé en** qu'il renferme une matière de référence ayant des teneurs différentes en ajouts de cervelle pour l'assurance de la qualité analytique.
